# EUROPÄISCHE PATENTSCHRIFT

(11) **EP 1 532 107 B1**
(45) Veröffentlichungstag und Bekanntmachung des Hinweises auf die Patenterteilung: **13.03.2013**
(21) Anmeldenummer: 03797208.0
(22) Anmeldetag: 24.07.2003
(51) Int. Cl.: C07C 265/14, C07C 263/10

(54) **Gasphasenphosgenierung bei moderaten Drücken**
Moderate-pressure gas phase phosgenation
Phosphogénation gazeuse à des pressions modérées

(30) Priorität: 20.08.2002 DE 10238995
(43) Veröffentlichungstag der Anmeldung: 25.05.2005
(73) Patentinhaber: BASF SE, 67056 Ludwigshafen (DE)
(72) Erfinder: WÖLFERT, Andreas, 74906 Bad Rappenau (DE); MÜLLER, Christian, 68167 Mannheim (DE); STROEFER, Eckhard, 68163 Mannheim (DE); WEBER, Markus, 67061 Ludwigshafen (DE); PFEFFINGER, Joachim, 67063 Ludwigshafen (DE); KNÖSCHE, Carsten, 67150 Niederkirchen (DE)
(86) Internationale Anmeldenummer: PCT/EP2003/008108
(87) Internationale Veröffentlichungsnummer: WO 2004/026813

(56) Entgegenhaltungen:
- EP-A- 0 150 435
- EP-A- 0 570 799
- EP-A- 0 593 334
- EP-A- 0 699 657
- WO-A-99/40059
- DE-A- 2 112 181

## Beschreibung

Die Erfindung betrifft Verfahren zur Herstellung von aromatischen Diisocyanaten durch Umsetzung von Phosgen mit Diaminen in der Gasphase, wobei die Reaktion in einem Reaktionsraum bei moderaten Drücken durchgeführt wird, d.h. der Druck in diesem Reaktionsraum beträgt mehr als 3 bar und weniger als 20 bar, wobei die Temperatur im Reaktionsraum mehr als 200°C bis weniger als 600°C beträgt .

Die Herstellung von organischen Isocyanaten aus den entsprechenden Aminen durch Phosgenierung in der Gasphase ist allgemein bekannt. Während die Phosgenierung von aliphatischen Aminen in der Gasphase bereits hinlänglich beschrieben ist, ist die großtechnische Phosgenierung von aromatischen Aminen in der Gasphase bisher noch nicht verwirklicht. Insbesondere treten hier Probleme durch Bildung von Feststoffen auf, welche die Misch- und Reaktionsvorrichtungen verstopfen und die Ausbeute vermindern. Außerdem ist bekannt, dass wegen der aromatischen Ringstruktur die Reaktivität von aromatischen Aminen mit Phosgen geringer ist, was zu schlechteren Raumzeitausbeuten führt.

Zur Verringerung dieser Probleme wurden mehrere Möglichkeiten vorgeschlagen. EP-A-570 799 beschreibt eine kontinuierliche Gasphasenphosgenierung für aromatische Amine, wobei die Umsetzung bei Temperaturen oberhalb des Siedepunktes des verwendeten Diamins erfolgt und die Vermischung der Reaktionspartner so eingestellt wird, dass eine mittlere Kontaktzeit von 0,5 bis 5 Sekunden und eine Abweichung von der mittleren Kontaktzeit von weniger als 6 % erreicht wird.

EP-A-593 334 beschreibt ein Verfahren zur Herstellung von aromatische Isocyanaten in der Gasphase, wobei ein Rohrreaktor verwendet wird, worin eine Vermischung der Edukte ohne bewegliches Rühren durch eine Einengung der Wände erreicht wird.

EP-A-699 657 offenbart ein Verfahren zur Herstellung von aromatischen Diisocyanaten in der Gasphase in einem Mischreaktor, wobei der Mischreaktor in zwei Zonen aufgeteilt wird, wovon die erste eine vollkommene Durchmischung der Edukte und die zweite eine kolbenartige Strömung gewährleistet.

Aufgabe der Erfindung war es, ein Verfahren bereit zu stellen, das eine technisch vorteilhafte Umsetzung, insbesondere im Hinblick auf eine hohe Raum-Zeit-Ausbeute und ein geringes Auftreten von störenden Feststoffen, von aromatischen Diaminen mit Phosgen in der Gasphase zu den entsprechenden Diisocyanaten gewährleistet.

Ferner war es Aufgabe der Erfindung, eine Produktionsanlage bereit zu stellen, mit der das erfindungsgemäße Verfahren vorteilhaft durchgeführt werden kann und die möglichst wenig der toxischen Substanz Phosgen enthält.

Die Aufgabe der Erfindung konnte unerwartet dadurch gelöst werden, dass die Gasphasenphosgenierung bei moderaten Drücken durchgeführt wird.

Gegenstand der Erfindung ist somit ein Verfahren zur Herstellung von aromatischen Diisocyanaten durch Umsetzung von Phosgen mit Diaminen in der Gasphase, dadurch gekennzeichnet, dass die Reaktion in einem Reaktionsraum durchgeführt wird, wobei der Druck in diesem Reaktionsraum mehr als 3 bar und weniger als 20 bar beträgt, wobei die Temperatur im Reaktionsraum mehr als 200°C bis weniger als 600°C beträgt.

Gegenstand der Erfindung ist ferner eine Produktionsanlage zur Herstellung von aromatischen Diisocyanaten durch Umsetzung von Phosgen mit Diaminen in der Gasphase bei einem Druck zwischen mehr als 3 bar und 25 bar, wobei die Produktionsanlage ein Verhältnis von Produktionskapazität zu Phosgen-Hold-Up von mehr als 3200 [Tonnen Diisocyanat pro Jahr / Kilogramm Phosgen] aufweist.

Für das erfindungsgemäße Verfahren kann ein beliebiges primäres aromatisches Diamin, das bevorzugt ohne Zersetzung in die Gasphase überführt werden kann, oder ein Gemisch aus zwei oder mehr solcher Amine eingesetzt werden. Bevorzugt werden beispielsweise Methylen-di(phenylamin) (einzelne Isomere und/oder Isomerengemisch), Toluylendiamin, R,S-1-Phenylethylamin, 1-Methyl-3-phenylpropylamin, 2,6-Xylidin, Naphthyldiamin und 3,3'-Diaminodiphenylsulfon. Besonders vorteilhaft kann das Verfahren zur Herstellung von Methylen-di(phenylisocyanat) (MDI) und Toluylendiisocyanat (TDI), insbesondere für Toluylendiisocyanat, angewandt werden. Die Erfindung umfasst nicht die Gasphasenphosgenierung von aliphatischen Diaminen.

Dem erfindungsgemäßen Verfahren kann ein zusätzliches Inertmedium beigesetzt werden. Bei dem Inertmedium handelt es sich um ein Medium, das bei der Reaktionstemperatur gasförmig im Reaktionsraum vorliegt und nicht mit den Edukten reagiert. Das Inertmedium wird im allgemeinen vor der Umsetzung mit Amin und/oder Phosgen vermischt. Beispielsweise können Stickstoff, Edelgase wie Helium oder Argon oder Aromaten wie Chlorbenzol, Dichlorbenzol oder Xylol verwendet werden. Bevorzugt wird Stickstoff als Inertmedium verwendet. Besonders bevorzugt ist Monochlorbenzol.

Im allgemeinen wird das Inertmedium in einer Menge eingesetzt, so dass das molare Verhältnis Inertmedium zu Diamin mehr als 2 bis 30, bevorzugt 2,5 bis 15 beträgt. Bevorzugt wird das Inertmedium zusammen mit dem Diamin in den Reaktionsraum eingeführt.

Dem erfindungsgemäßen Verfahren kann ein Lösungsmittel zugegeben werden. Das Lösungsmittel wird im Gegensatz zum Inertmedium im allgemeinen erst nach der Umsetzung der Edukte im Reaktionsraum, d.h. bevorzugt in der Aufarbeitungsstufe zugegeben. Bevorzugt liegt das Lösungsmittel in flüssiger Form vor. Es handelt sich bei dem Lösungsmittel um Stoffe, welche inert gegenüber den Edukten und Produkten des erfindungsgemäßen Verfahrens sind. Bevorzugt sollte das Lösungsmittel gute, d.h. selektive Löseeigenschaften für das herzustellende Isocyanat aufweisen.

In einer bevorzugten Ausführungsform handelt es sich bei dem Inertmedium und bei dem Lösungsmittel um die selbe Verbindung, besonders bevorzugt wird in diesem Fall Monochlorbenzol verwendet.

Die Umsetzung von Phosgen mit Diamin erfolgt in einem Reaktionsraum, der im allgemeinen in einem Reaktor angeordnet ist, d.h. unter Reaktionsraum wird der Raum verstanden, wo die Umsetzung der Edukte erfolgt, unter Reaktor wird die technische Vorrichtung verstanden, die den Reaktionsraum enthält. Hierbei kann es sich um alle üblichen, aus dem Stand der Technik bekannten Reaktionsräume handeln, die zur nicht katalytischen, einphasigen Gasreaktion, bevorzugt zur kontinuierlichen nicht katalytischen, einphasigen Gasreaktion, geeignet sind und die den geforderten moderaten Drücken standhalten. Geeignete Materialien für den Kontakt mit dem Reaktionsgemisch sind z.B. Metalle, wie Stahl, Tantal, Silber oder Kupfer, Glas, Keramik, Emaille oder homogenen oder heterogenen Gemischen daraus. Bevorzugt werden Stahlreaktoren verwendet. Die Wände des Reaktors können glatt oder profiliert sein. Als Profile eignen sich beispielsweise Ritzen oder Wellen.

Es können im allgemeinen die aus dem Stand der Technik bekannten Reaktorbautypen verwendet werden. Bevorzugt verwendet werden Rohrreaktoren.

Ebenfalls ist es möglich im wesentlichen quaderförmige Reaktionsräume, bevorzugt Plattenreaktoren bzw. Plattenreaktionsräume zu verwenden. Ein besonders bevorzugter Plattenreaktor weist ein Verhältnis von Breite zu Höhe von mindestens 2:1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionsraums ab und ist prinzipiell nicht begrenzt. Technisch sinnvoll haben sich Reaktionsräume mit einem Verhältnis von Breite zu Höhe bis maximal 5000 : 1, bevorzugt 1000 : 1 erwiesen.

Im erfindungsgemäßen Verfahren erfolgt die Vermischung der Reaktanten in einer Mischeinrichtung, die sich durch eine hohe Scherung des durch die Mischeinrichtung geführten Reaktionsstromes auszeichnet. Bevorzugt werden als Mischeinrichtung eine statische Mischeinrichtung oder eine Mischdüse verwendet, die dem Reaktor vorangestellt ist. Besonders bevorzugt wird eine Mischdüse verwendet.

Die Umsetzung von Phosgen mit Diamin im Reaktionsraum erfolgt bei Absolutdrücken von mehr als 3 bar bis weniger als 20 bar, bevorzugt zwischen 3,5 bar und 15 bar, besonders bevorzugt zwischen 4 bar und 12 bar, insbesondere von 5 bis 12 bar.

Im allgemeinen ist der Druck in den Zuleitungen zur Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 1000 mbar, besonders bevorzugt von 30 bis 200 mbar höher als im Reaktionsraum.

Im allgemeinen ist der Druck in der Aufarbeitungsvorrichtung niedriger als im Reaktionsraum. Bevorzugt ist der Druck um 50 bis 500 mbar, besonders bevorzugt 80 bis 150 mbar, niedriger als im Reaktionsraum.

Gemäß dem erfindungsgemäßen Verfahren erfolgt die Umsetzung von Phosgen mit Diamin in der Gasphase. Unter Umsetzung in der Gasphase ist zu verstehen, dass die Eduktströme im gasförmigen Zustand miteinander reagieren.

Bei dem erfindungsgemäßen Verfahren wird die Temperatur im Reaktionsraum so gewählt wird, dass sie unterhalb der Siedetemperatur des eingesetzten Diamins, bezogen auf die im Reaktionsraum herrschenden Druckverhältnisse, liegt. Je nach eingesetztem Amin und eingestellten Druck ergibt sich üblicherweise eine vorteilhafte Temperatur im Reaktionsraum von mehr als 200°C bis weniger als 600°C, bevorzugt von 280°C bis 400°C. Zur Durchführung des erfindungsgemäßen Verfahrens kann es vorteilhaft sein, die Ströme der Reaktanten vor dem Vermischen vorzuwärmen, üblicherweise auf Temperaturen von 100 bis 600°C, bevorzugt von 200 bis 400°C.

Die mittlere Kontaktzeit des Umsetzungsgemisches im erfindungsgemäßen Verfahren beträgt im allgemeinen zwischen 0,1 Sekunden und weniger als 5 Sekunden, bevorzugt von mehr als 0,5 Sekunden bis weniger als 3 Sekunden, besonders bevorzugt von mehr als 0,6 Sekunden bis weniger als 1,5 Sekunden. Unter mittlerer Kontaktzeit wird die Zeitspanne vom Beginn der Vermischung der Edukte bis zum Verlassen des Reaktionsraumes verstanden.

In einer bevorzugten Ausführungsform werden die Abmessungen des Reaktionsraums und die Strömungsgeschwindigkeiten so bemessen, dass eine turbulente Strömung, d.h. eine Strömung mit einer Reynolds-Zahl von mindestens 2300, bevorzugt mindestens 2700, vorliegt, wobei die Reynolds-Zahl mit dem hydraulischen Durchmesser des Reaktionsraumes gebildet wird. Bevorzugt durchlaufen die gasförmigen Reaktionspartner den Reaktionsraum mit einer Strömungsgeschwindigkeit von 3 bis 180 Meter/Sekunde, bevorzugt von 10 bis 100 Meter/Sekunde. Durch die turbulente Strömung werden eine enge Verweilzeit und eine gute Vermischung erreicht. Maßnahmen, wie beispielsweise die in EP-A-593 334 beschriebene Verengung, die zudem verstopfungsanfällig ist, sind nicht notwendig.

Im erfindungsgemäßen Verfahren beträgt üblicherweise das molare Verhältnis von Phosgen zu eingesetztem Diamin beträgt im allgemeinen 2 : 1 bis 30 : 1, bevorzugt 2,5 : 1 bis 20 : 1, besonders bevorzugt 3 : 1 bis 15 : 1.

In einer bevorzugten Ausführungsform werden die Umsetzungsbedingungen so gewählt, dass das Reaktionsgas am Austritt aus dem Reaktionsraum eine Phosgenkonzentration von mehr als 25 mol/m³, bevorzugt von 30 bis 50 mol/m³, aufweist. Weiterhin liegt am Austritt aus dem Reaktionsraum im allgemeinen eine Inertmediumskonzentration von mehr als 25 mol/m³, bevorzugt von 30 bis 100 mol/m³ vor.

In einer besonders bevorzugten Ausführungsform werden die Umsetzungsbedingungen so gewählt, dass das Reaktionsgas am Austritt aus dem Reaktionsraum eine Phosgenkonzentration von mehr als 25 mol/m³, insbesondere von 30 bis 50 mol/m³, und zugleich eine Inertmediumskonzentration von mehr als 25 mol/m³, insbesondere von 30 bis 100 mol/m³, besitzt.

Das Reaktionsvolumen wird üblicherweise über seine Außenfläche temperiert. Um Produktionsanlagen mit hoher Anlagenkapazität zu bauen, können mehrere Reaktorrohre parallel geschalten werden.

Das erfindungsgemäße Verfahren wird bevorzugt einstufig durchgeführt. Darunter ist zu verstehen, dass die Vermischung und Umsetzung der Edukte in einem Schritt und in einem Temperaturbereich, bevorzugt in dem vorstehend genannten Temperaturbereich, erfolgt. Ferner wird das erfindungsgemäße Verfahren bevorzugt kontinuierlich durchgeführt.

Nach der Reaktion wird im allgemeinen das gasförmige Umsetzungsgemisch bevorzugt bei Temperaturen größer 150°C mit einem Lösungsmittel gewaschen. Als Lösungsmittel sind bevorzugt Kohlenwasserstoffe, die gegebenenfalls mit Halogenatomen substituiert sind, geeignet, wie beispielsweise Chlorbenzol, Dichlorbenzol, und Toluol. Als Lösungsmittel wird besonders bevorzugt Monochlorbenzol eingesetzt. Bei der Wäsche wird das Isocyanat selektiv in die Waschlösung übergeführt. Anschließend werden das verbleibende Gas und die erhaltene Waschlösung bevorzugt mittels Rektifikation in Isocyanat(e), Lösungsmittel, Phosgen und Chlorwasserstoff aufgetrennt. Geringe Mengen von Nebenprodukten, die im Isocyanat(e) verbleiben, können mittels zusätzlicher Rektifikation oder auch Kristallisation vom erwünschten Isocyanat(e) getrennt werden.

In einer bevorzugten Ausführungsform wird das erfindungsgemäße Verfahren in einer Produktionsanlage durchgeführt, wobei der Phosgen-Hold-Up im Reaktionsraum zur Umsetzung von Amin mit Phosgen der Anlage weniger als 100 kg, bevorzugt weniger als 60 kg, besonders bevorzugt weniger als 40 kg beträgt. Unter Phosgen-Hold-Up im Reaktionsraum zur Umsetzung von Amin mit Phosgen ist hierbei die bei Normalbetrieb im Reaktionsraum zur Umsetzung von Amin mit Phosgen enthaltene Phosgenmasse in kg zu verstehen.

Gegenstand der Erfindung ist eine Produktionsanlage, die zur Durchführung des erfindungsgemäßen Verfahrens geeignet ist, d.h. eine Produktionsanlage zur Herstellung von aromatischen Diisocyanaten durch Umsetzung von Phosgen mit Diaminen in der Gasphase, bevorzugt bei einem Absolutdruck im Reaktionsraum, in dem die Umsetzung stattfindet, von mehr als 3 bar und weniger als 20 bar, wobei die Temperatur im Reaktionsraum mehr als 200°C bis weniger als 600°C beträgt.

In einer bevorzugten Ausführungsform handelt es sich hierbei um eine Produktionsanlage, die 50000 bis 500000 Tonnen an gewünschtem Diisocyanat pro Jahr, mehr bevorzugt 100000 bis 300000 Tonnen Diisocyanat pro Jahr und besonders bevorzugt 150000 bis 250000 Tonnen Diisocyanat pro Jahr produziert.

Die erfindungsgemäße Produktionsanlage enthält Vorlagenvorrichtungen für Diamin und Phosgen, eine Mischvorrichtung, einen oder mehrere Reaktoren und eine Aufarbeitungsvorrichtung und gegebenenfalls eine Reinigungsvorrichtung.

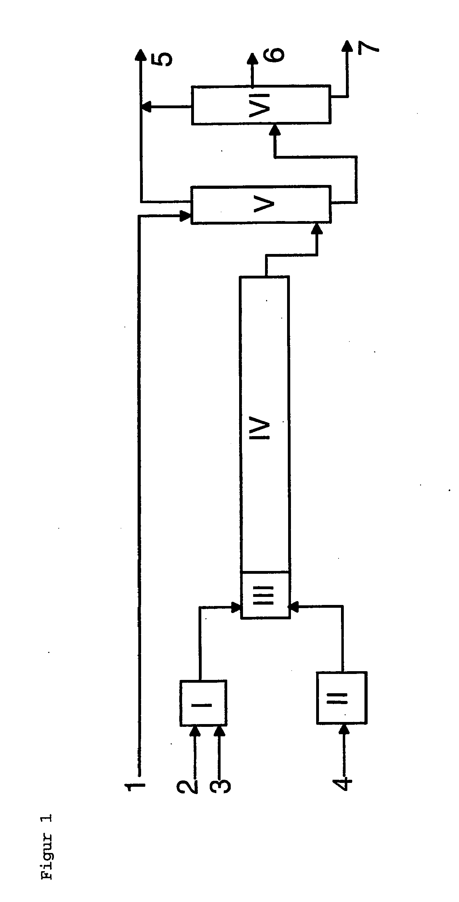

Ein Beispiel für eine erfindungsgemäße Produktionsanlage ist in Figur 1 abgebildet.

In Figur 1 bedeutet:
I Aminvorlage
II Phosgenvorlage
III Mischeinheit
IV Reaktor
V Aufarbeitungsvorrichtung mit Quench
VI Reinigungsvorrichtung
   1 Zufuhr Lösungsmittel
   2 Zufuhr Amin
   3 Zufuhr Inertmedium
   4 Zufuhr Phosgen
   5 Austrag HCl und/oder Phosgen und/oder Inertmedium
   6 Austrag Inertmedium und/oder Lösungsmittel
   7 Austrag Isocyanat und/oder Lösungsmittel

In der Aminvorlage wird das Diamin zusammen mit einem Inertmedium als Trägergas wie beispielsweise Stickstoff, in die Gasphase überführt und in die Mischeinheit eingespeist. Ebenfalls wird Phosgen aus der Phosgenvorlage in die Gasphase übergeführt und in die Mischeinheit geleitet. Nach dem Vermischen in der Mischeinheit, die beispielsweise aus einer Düse oder einem statischen Mischer bestehen kann, wird das gasförmige Gemisch aus Phosgen, Amin und Inertmedium in den Reaktor überführt, wobei der Reaktor den Reaktionsraum enthält.

In einer bevorzugten Ausführungsform besteht der Reaktor aus einem Bündel an Reaktoren. In einer möglichen Ausführungsform muss es sich bei der Mischeinheit nicht um eine eigenständige Vorrichtung handeln, vielmehr kann es vorteilhaft sein, die Mischeinheit in den Reaktor zu integrieren. Ein Beispiel einer integrierten Einheit aus Mischeinheit und Reaktor stellt ein Rohrreaktor mit angeflanschten Düsen dar.

Nachdem das Reaktionsgemisch im Reaktionsraum umgesetzt wurde, gelangt es in die Aufarbeitungsvorrichtung mit Quench. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Als inertes Lösungsmittel sind bevorzugt Kohlenwasserstoffe, die gegebenenfalls mit Halogenatomen substituiert sind, geeignet, wie beispielsweise Chlorbenzol, Dichlorbenzol, und Toluol. Besonders bevorzugt wird dabei die Temperatur des inerten Lösungsmittel oberhalb der Zersetzungstemperatur des zum Amin gehörigen Carbamylchlorids gehalten.

In der anschließenden optionalen Reinigungsstufe wird das Isocyanat, bevorzugt durch Destillation, vom Lösungsmittel abgetrennt. Ebenfalls kann hier noch die Abtrennung von restlichen Verunreinigungen, umfassend Chlorwasserstoff, Inertmedium und/oder Phosgen, erfolgen.

Die erfindungsgemäße Produktionsanlage ist so aufgebaut, dass das Verhältnis von Produktionskapazität zu Phosgen-Hold-Up mehr als 3200 [Tonnen Diisocyanat pro Jahr / Kilogramm Phosgen], bevorzugt mehr als 4000, besonders bevorzugt mehr als 5000 aufweist. Die Obergrenze des Verhältnisses von maximaler Produktionskapazität zu Phosgen-Hold-Up ist im allgemeinen nicht begrenzt, es hat sich jedoch ein Wert von 20000, bevorzugt von 10000 als sinnvoll erwiesen.

Die Erfindung soll durch nachfolgende Beispiele veranschaulicht werden:

### Beispiel 1

Ein auf 320°C erwärmter Gasstrom aus Monochlorbenzol und Toluylendiamin, der zu 74 Massenprozent aus Monochlorbenzol und zu 26 Massenprozent aus Toluylendiamin bestand, und der einen Massenstrom von 30 g/min besaß, wurde in einem 2 Meter langen Strömungsrohr von 8 mm Innendurchmesser mit einem auf 300°C vorgewärmten Phosgenstrom mit einem Massenstrom von 64 g/min bei einem Druck von 10 bar nach Vermischung in einer Mischdüse zur Reaktion gebracht. Dabei wurde die Strömungsrohrwand auf 380°C temperiert. Das Strömungsrohr verlassende Gemisch hatte eine Temperatur von 384°C und wurde in 160°C warmen Monochlorbenzol gequencht, um das entstandene Isocyanat aus der Gasphase auszuwaschen. Nach dem destillativen Abtrennen von Phosgenresten aus der Quenchphase wurde die Probe gaschromatographisch analysiert. Die erreichte Toluylendiisocyanat-Ausbeute betrug ca. 99,2 %. Die Phosgenkonzentration am Austritt aus dem Strömungsrohr betrug ca. 90 mol/m³. Die Monochlorbenzolkonzentration am Austritt aus dem Strömungsrohr betrug ca. 35 mol/ m³.

### Beispiel 2

Ein auf 380°C erwärmter Gasstrom aus Monochlorbenzol und Methylen-di(phenylamin), der zu 84 Massenprozent aus Monochlorbenzol und zu 16 Massenprozent aus Methylen-di(phenylamin) bestand, und der einen Massenstrom von 54,4 g/min besaß, wurde in einem 1 Meter langen Strömungsrohr von 8 mm Innendurchmesser mit einem auf 380°C vorgewärmten Phosgenstrom mit einem Massenstrom von 44,4 g/min bei einem Druck von 5 bar nach Vermischung in einer Mischdüse zur Reaktion gebracht. Dabei wurde die Reaktorwand auf 380°C temperiert. Das Strömungsrohr verlassende Gemisch hatte eine Temperatur von 385°C und wurde in 160°C warmen Monochlorbenzol gequencht, um das entstandene Isocyanat aus der Gasphase auszuwaschen. Nach dem destillativen Abtrennen von Phosgenresten aus der Quenchphase wurde die Probe gaschromatographisch analysiert. Die erreichte Methylen-di(phenylisocyanat)-Ausbeute betrug ca. 99,3 %. Die Phosgenkonzentration am Austritt aus dem Strömungsrohr betrug ca. 33 mol/m³. Die Monochlorbenzolkonzentration am Austritt aus dem Strömungsrohr betrug ca. 38 mol/ m³.

## Patentansprüche

1. Verfahren zur Herstellung von aromatischen Diisocyanaten durch Umsetzung von Phosgen mit Diaminen in der Gasphase, **dadurch gekennzeichnet, dass** die Reaktion in einem Reaktionsraum durchgeführt wird, wobei der Druck in diesem Reaktionsraum mehr als 3 bar und weniger als 20 bar beträgt und die Temperatur im Reaktionsraum mehr als 200°C bis weniger als 600°C beträgt.

2. Verfahren nach Anspruch 1, **dadurch gekennzeichnet, dass** die Temperatur im Reaktionsraum so gewählt wird, dass sie unterhalb der Siedetemperatur des eingesetzten Diamins, bezogen auf die im Reaktionsraum herrschenden Druckverhältnisse, liegt.

3. Verfahren nach einem der Ansprüche 1 bis 2, **dadurch gekennzeichnet, dass** neben Diamin und Phosgen zusätzlich ein Inertmedium dem Reaktionsraum zugeführt wird, wobei eine Inertmediumskonzentration von mehr als 25 mol/m³ am Austritt aus dem Reaktionsraum vorliegt.

4. Verfahren nach einem der Ansprüche 1 bis 3, **dadurch gekennzeichnet, dass** im Reaktionsgas am Austritt aus dem Reaktionsraum eine Konzentration an Phosgen von mehr als 25 mol/m³ vorliegt.

5. Verfahren nach einem der Ansprüche 1 bis 4, **dadurch gekennzeichnet, dass** es sich bei dem Verfahren um ein kontinuierliches Verfahren handelt.

6. Verfahren nach einem der Ansprüche 1 bis 5, **dadurch gekennzeichnet, dass** das Verfahren in einer Produktionsanlage durchgeführt wird, wobei der Phosgen-Hold-Up im Reaktionsraum zur Umsetzung des aromatischen Diamins mit Phosgen der Anlage weniger als 100 kg beträgt.

7. Produktionsanlage zur Herstellung von aromatischen Diisocyanaten durch Umsetzung von Phosgen mit Diaminen in der Gasphase bei einem Druck von mehr als 3 und weniger als 20 bar, wobei die Temperatur im Reaktionsraum mehr als 200°C bis weniger als 600°C beträgt, und wobei die Produktionsanlage ein Verhältnis von Produktions-kapazität zu Phosgen-Hold-Up von mehr als 3200 [Tonnen Diisocyanat pro Jahr/Kilogramm Phosgen] aufweist.

8. Produktionsanlagen gemäß Anspruch 7, **dadurch gekennzeichnet, dass** die Produktionskapazität mehr als 50000 Tonnen Diisocyanat pro Jahr beträgt.

## Claims

1. A process for preparing aromatic diisocyanates by reaction of phosgene with diamines in the gas phase, wherein the reaction is carried out in a reaction zone in which the pressure is more than 3 bar and less than 20 bar and the temperature in the reaction zone is from more than 200□C to less than 600°C.

2. The process according to claim 1, wherein the temperature in the reaction zone is selected so that it is below the boiling point of the diamine used under the pressure conditions prevailing in the reaction zone.

3. The process according to claim 1 or 2, wherein an inert medium is fed into the reaction zone in addition to diamine and phosgene in such an amount that the concentration of inert medium at the outlet from the reaction zone is more than 25 mol/m³.

4. The process according to any of claims 1 to 3, wherein the concentration of phosgene in the reaction gas at the outlet from the reaction zone is more than 25 mol/m³.

5. The process according to any of claims 1 to 4 which is carried out continuously.

6. The process according to any of claims 1 to 5 which is carried out in a production plant in which the phosgene holdup in the reaction zone for the reaction of the aromatic diamine with phosgene in the plant is less then 100 kg.

7. A production plant for preparing aromatic diisocyanates by reaction of phosgene with diamines in the gas phase at a pressure of more than 3 bar and less than 20 bar, wherein the temperature in the reaction zone is from more than 200°C to less than 600°C the production plant has a ratio of production capacity to phosgene holdup of more than 3200 [metric tons of diisocyanate per year/kilogram of phosgene].

8. The production plant according to claim 7 which has a production capacity of more than 50 000 metric tons of diisocyanate per year.

## Revendications

1. Procédé pour la production de diisocyanates aromatiques par mise en réaction de phosgène avec des diamines en phase gazeuse, **caractérisé en ce qu'**on effectue la réaction dans une chambre de réaction, la pression dans cette chambre de réaction étant supérieure à 3 bars et inférieure à 20 bars et la température dans la chambre de réaction valant plus de 200 °C à moins de 600 °C.

2. Procédé selon la revendication 1, **caractérisé en ce que** la température dans la chambre de réaction est choisie de manière à se situer au-dessous de la température d'ébullition de la diamine utilisée, sur la base des conditions de pression régnant dans la chambre de réaction.

3. Procédé selon la revendication 1 ou 2, **caractérisé en ce qu'**en plus de diamine et de phosgène, on envoie en outre à la chambre de réaction un milieu inerte, une concentration de milieu inerte de plus de 25 moles/m³ étant présente à la sortie de la chambre de réaction.

4. Procédé selon l'une quelconque des revendications 1 à 3, **caractérisé en ce qu'**une concentration de phosgène de plus 25 moles/m³ est présente dans le gaz de réaction à la sortie de la chambre de réaction.

5. Procédé selon l'une quelconque des revendications 1 à 4, **caractérisé en ce que** le procédé consiste en un procédé continu.

6. Procédé selon l'une quelconque des revendications 1 à 5, **caractérisé en ce qu'**on effectue le procédé dans une unité de production, la rétention de phosgène dans la chambre de réaction destinée à la réaction de la diamine aromatique avec le phosgène de l'unité étant inférieure à 100 kg.

7. Unité de production pour la production de diisocyanates aromatiques par mise en réaction de phosgène avec des diamines en phase gazeuse sous une pression supérieure à 3 et inférieure à 20 bars, la température dans la chambre de réaction valant de plus de 200 °C à moins de 600 °C, et l'unité de production présentant un rapport de la capacité de production à la retenue de phosgène de plus de 3 200 (tonnes de diisocyanate par an/kilogramme de phosgène).

8. Unités de production selon la revendication 7, **caractérisées en ce que** la capacité de production est de plus de 50 000 tonnes de diisocyanate par an.
